# EUROPEAN PATENT APPLICATION

(11) **EP 4 596 710 A2**
(43) Date of publication of application: **06.08.2025**
(21) Application number: 25183432.1
(22) Date of filing: 20.05.2022
(51) Int. Cl.: C12Q 1/68

(54) **METHODS AND CONSTRUCTS FOR LOCATING AND PROFILING SINGLE CELLS IN A BIOLOGICAL SAMPLE**

(30) Priority: 21.05.2021 EP 21175329
(62) Divisional of application: 22726267.2
(71) Applicant: SCellex Oy, 00290 Helsinki (FI)
(72) Inventor: SAAVALAINEN, Päivi, 00290 Helsinki (FI)
(74) Representative: Laine IP Oy

(57) **Abstract**

The present invention generally relates to preparation of biological samples for next generation sequencing. Particularly, the invention utilizes a combination of sequence barcodes and visible bead features such as fluorescent label combinations for identification of mRNA products originating from a single cell and for locating said single cell in the sample. To achieve this effect, the present invention provides a novel construct comprising a bead coupled to such visible feature, wherein said bead is further coupled to a first oligonucleotide by a cleavable, preferably photocleavable, linker, said first oligonucleotide comprising a) an amplification handle sequence, b) a barcode sequence specific to said bead feature and c) an anchor or capture sequence, preferably a poly-A sequence, for hybridizing to a complementary sequence.

## Description

### FIELD OF THE INVENTION

The present invention generally relates to preparation of biological samples for next generation sequencing. Particularly, the invention utilizes a random combination of microbeads with microscopically detectable or otherwise visible labels coupled to sequence barcode for spatial labelling and identification of mRNA or DNA products originating from a single position and for locating said single position in the sample slide.

### BACKGROUND OF THE INVENTION

In the complex cellular environment, cells of a tissue often display multiple phenotypes, which is reflected in RNA expression profiles of the cells. In the prior art, RNA expression profiling has been applied to single cells in suspensions, typically utilizing microwells or emulsion droplets where the single cells are encapsulated together with microbeads coated with unique cell barcode sequence and poly-T which capture the poly-A mRNA of the lysing cell, labelling the 3' end of the mRNA with the cell barcode sequence in reverse transcription reaction and cDNA synthesis (see e.g. Macosko et al 2015 and WO2016040476 by the Broad Institute Inc.). After sequencing the mRNA transcripts derived from same original cell can be grouped together based on the cell barcode, and separate gene expression profiles of all cells generated. The current cell barcoding beads, however, are targeted only to cells in suspension.

To understand the tissue structure, cell functions and cell-to-cell interactions within the tissues, there is a need to link RNA expression data with the cell image and spatial information gained from examination of cells or tissue sections with single cell resolution and high throughput manner. WO2021046232 discloses a system based on optically readable barcodes for characterization of molecular interactions of single cells. The system is relying on the use of oligonucleotide probes with multiple fluorescent labels. Improved methods are still in need.

### SUMMARY OF THE INVENTION

The present invention as defined in the appended claims is based on a novel design of microbeads with visible labels coupled with label specific barcodes that make it possible to link the obtained mRNA or DNA sequencing data to spatial coordinates of the cells in a biological sample profiled for RNA expression.

One object of the invention is to provide construct for detecting nucleic acid targets, said construct comprising a bead, preferably a magnetic bead, wherein said bead comprises one or several visually detectable features and/or said bead is coupled to one or several visually detectable entities, and wherein said bead comprises an oligonucleotide, and said oligonucleotide comprises a) an amplification handle sequence, b) a barcode sequence specific to one or several of said visually detectable features and/or entities and c) a 3' terminal capture or anchor sequence, and wherein said oligonucleotide is arranged to be detachable from said bead. Preferably, said terminal capture or anchor sequence comprises a poly-A sequence for hybridizing to a complementary poly-T sequence on commercially available cell barcoding beads.

Alternatively, also the cell barcoding sequence can be placed on the same construct with visible labels.

Another object of the invention is to provide a composition comprising several constructs as defined above preferably with a mixture of distinct visually detectable features or entities.

Another object of the invention is to provide a well plate or chip comprising the composition according to the present invention loaded to the wells of said plate or chip.

Another object of the invention is to provide a kit comprising the well plate or chip as defined above and a computer readable entity comprising a file of a photograph or scan of said plate or chip with the composition of the present invention loaded to the wells of said plate or chip.

Another object of the invention is to provide a use of said construct or composition for identifying desired nucleic acid targets or mRNA sequencing products originating from a single cell or position in biological sample.

Another object of invention is to provide a method for profiling biological samples on a single cell level, the method comprising the steps of:
- loading the construct or the composition of the present invention with a biological sample together or sequentially in any order to a substrate comprising wells,
- photographing or scanning the wells in said substrate with a microscope, preferably a light microscope or a fluorescence microscope, before and/or after loading the biological sample to said substrate; and
- optionally determining the location or coordinate of a specific well having a distinct visually detectable feature and/or entity or combination thereof in the loaded substrate.

### BRIEF DESCRIPTION OF THE DRAWINGS

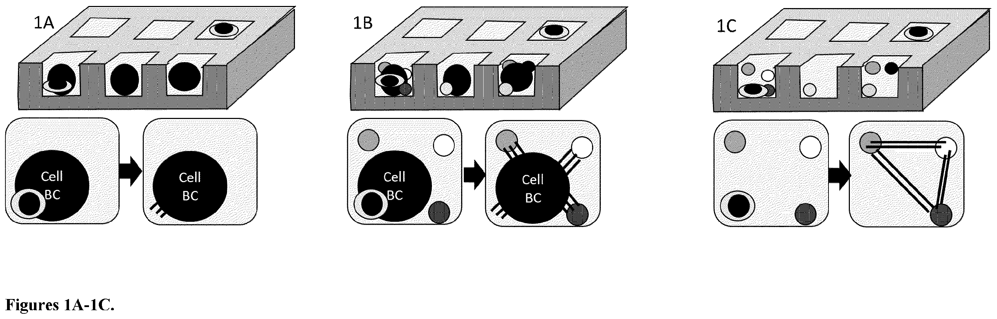

**Figure 1****.** A. Current methods for single cell RNAseq using random loading of Cell barcoding (CellBC) beads to wells or droplets. The unique bead specific cell barcode and polyT sequences on bead oligos capture and tag the polyA RNAs from the lysing cell. Spatial coordination between image and data is not, however, possible. B. New method with Colour barcoding (ColourBC) microbeads jointly with CellBC beads provides spatial coordinates for the data and image. Multiple differently coloured ColourBC beads are randomly loaded to wells or droplets, each with cleavable polyA tailed colour tagging oligos that will bind to the CellBC bead along with RNA from the cell, and links the data to spatial locations. C. Alternative way of spatial barcoding with ColourBC beads without CellBC beads. Both CellBC and ColourBC oligos are on the same ColourBC beads, the ColourBC oligos with e.g. photocleavable linker. After loaded in multiple beads per wells with cells, the ColourBC oligos are released from the beads and captured by the complementary sequences of the CellBC oligos in all beads in the well. The network of ColourBC and CellBC combination is unique for each well and RNA molecules tagged also by the CellBC sequences can be linked to the spatial location based on the microscope scan image.

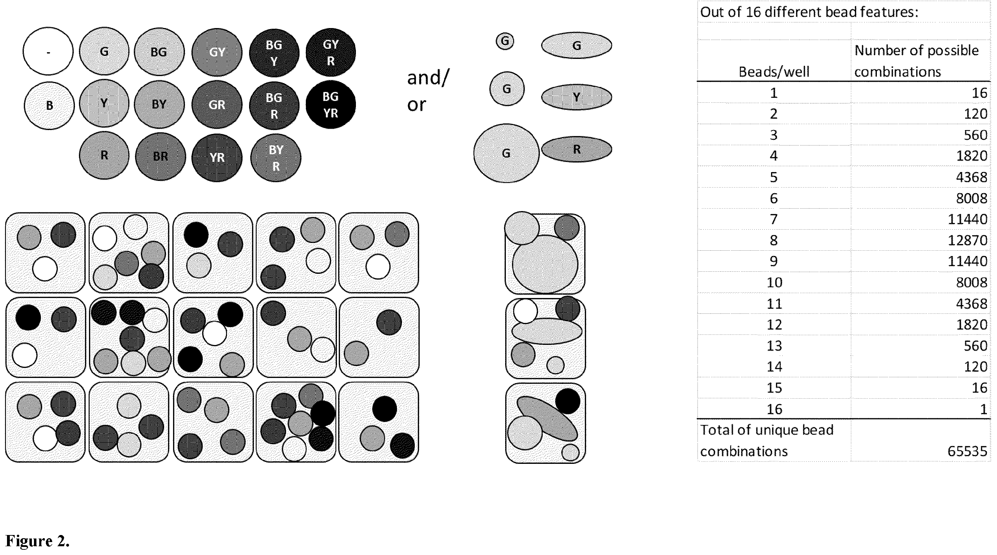

**Figure 2****.** Example of 16 different Colour BC microbeads created from four fluorescently labelled oligos, blue (B), green (G), yellow (Y), red (R), and 0, 1, 2, 3 and 4 colour combinations of them give rise to 16 different types of microbeads. When the mix of these 16 bead types are loaded randomly to the chip wells, with multiple beads fitting per well, they can form in 1 out of 65535 different combinations per each well according to permutation calculation, making the wells highly unique. Additional colours (fluorescent and non-fluorescent), different bead sizes and shapes and the colour labels attached also to the Cell BC beads, will increase the number of possible combinations further. Number of possible combinations was calculated with the tool at https://stattrek.com/online-calculator/combinations-permutations.aspx.

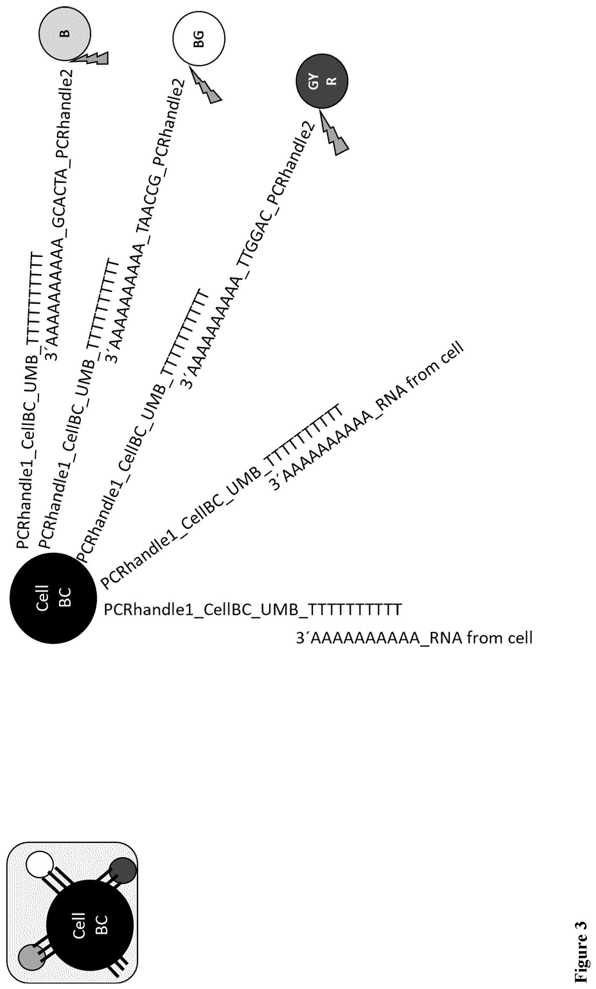

**Figure 3****.** Commercially available CellBC barcoding beads have millions of copies of a unique CellBC sequence on their surface oligo, created typically with split and pool synthesis, e.g. in DropSeq method a stretch of random 12 nucleotide barcode gives rise to nearly 17 million different barcodes and therefore 17 million different microbeads (Macosko et al, Cell 2015 May 21;161(5):1202-1214). The polyT stretch on the oligos will capture and hybridize polyA tailed RNA from the cells from tissue section. The CellBC beads look identical under the microscope. In the invention, random number of ColourBC microbeads from a mix of 16 different types will be loaded jointly with CellBC beads into the microwells and their synthetic, photocleavable polyA tailed oligo will also get the same CellBC sequence as the RNAs of the cell in the same microwell.

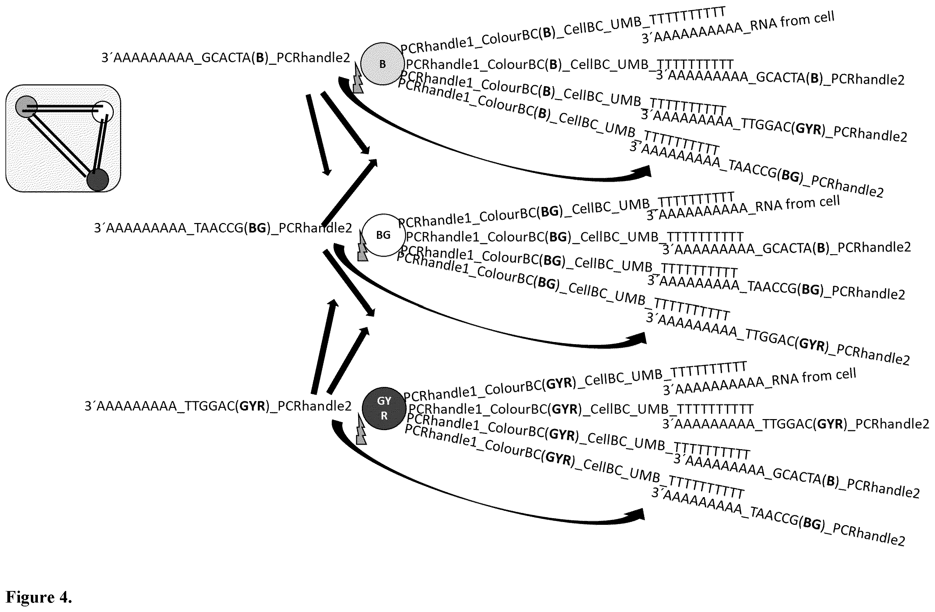

**Figure 4****.** Alternative way to use the ColourBC coordinates without separate CellBC beads. Coloured or otherwise featured beads carry each two types of oligos with the corresponding ColourBC, and at least one of the oligos carry also a CellBC unique to each bead as well as optional unique molecular barcode (UMB). One way to use the principle is shown in this figure: A random set of 3 bead types (out of e.g. 16) is in a microwell, and their synthetic, (photo)cleavable polyA ending oligos with ColourBCs are released by UV light or chemically, microwell contents are mixed with magnet and the released oligos will hybridize to the poly-T oligos of all 3 beads in the well, linking their unique CellBC's to the set of all ColourBCs present in the microwell. The cellular RNA will also get the CellBC's of the 3 beads, and sequence data from these mapped 3 CellBC's will be pooled together. In addition to the presented oligos, similar networks of ColourBCs and CellBC can be formed with other sites of library structures: using random primer (instead of synthetic poly-A) linked to ColourBC and PCRhandle2, and once released, using that for 2nd strand synthesis of the bead bound to first strand cDNA. Yet another way to use the principle is to place the CellBC and ColourBC elements to the Illumina index read positions in indexing primers, and perform the tagmentation and indexing PCR within the sealed microwells, with such primers released from the random mix of the Colour barcoded beads in each microwell.

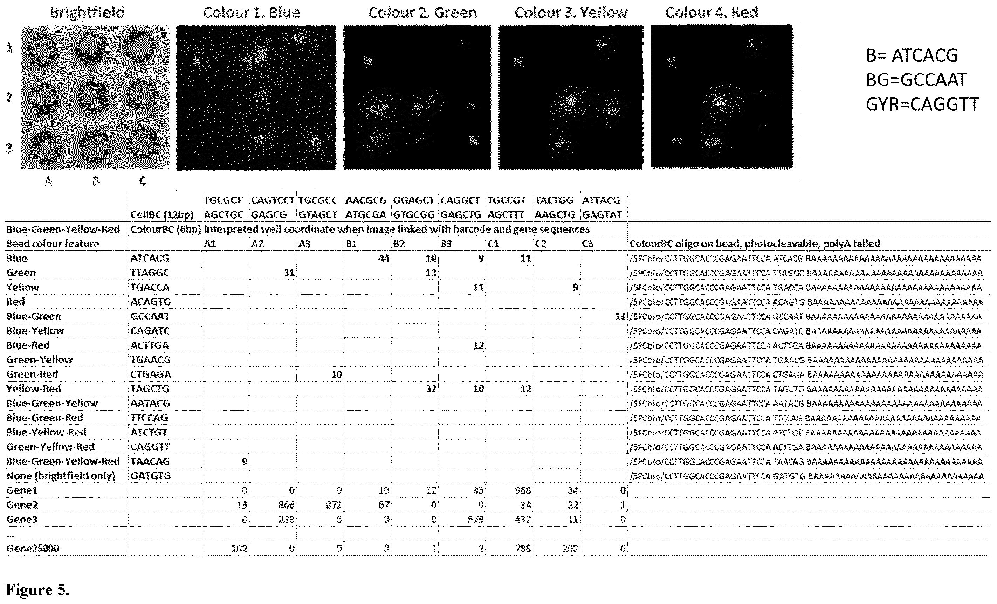

**Figure 5****.** Microwells with ColourBC microbeads are scanned with fluorescence microscope, stitched digital image saved and microbead combinations in each microwell are recorded by image analysis programs. After loading of CellBC beads the polyA RNA from lysed cells or tissue sections are hybridized to the CellBC barcode oligos, along with the synthetic polyA tailed ColourBC oligos released by UV from the ColourBC microbeads. After reverse transcription, cDNA amplification and sequencing library preparation and sequencing, the CellBC sequences are determined from the reads. After read alignment against target genome the counts of the gene reads along with the read counts of the ColourBC sequences are ordered into a matrix for each CellBC. Positional microwell coordinate is interpret for each CellBC by linking the ColourBC read information to the analysed microscope image of each microwell.

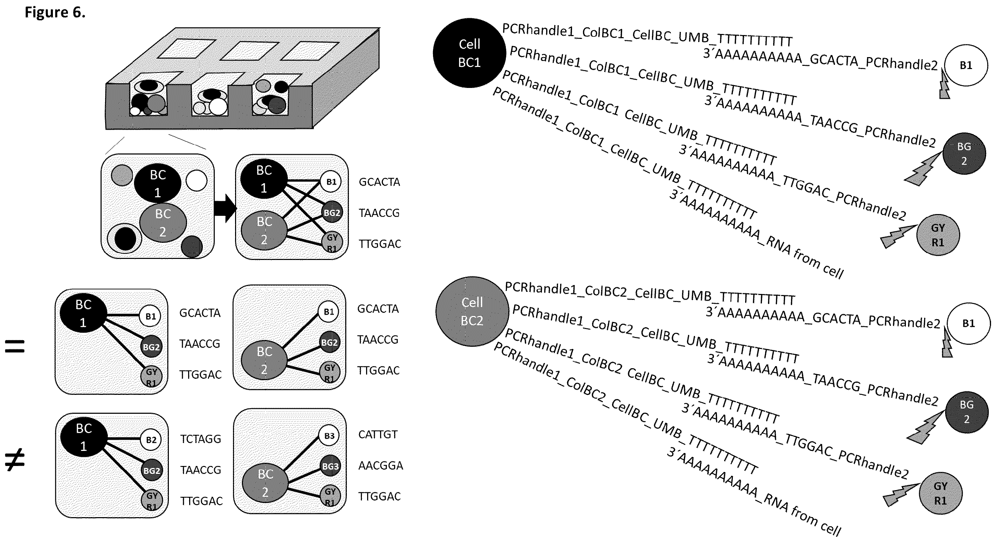

**Figure 6****.** Alternative way to use the ColourBC beads together with CellBC beads that are coloured as well. In this method, the CellBC beads present additional size and/or colour features distinguishable from the ColourBC beads and carry the feature matching sequence information incorporated into the CellBC oligo. As the CellBC beads have therefore multiple features, this method yields in higher number of possible bead combinations than the method of Figure 1 and has a simpler bead barcode synthesis process than the method of Figure 4. Like in the method of Figure 3, this requires that at least one CellBC bead and one ColourBC bead enter a well. It is recommended to have CellBC beads larger in size than the ColourBC beads, to maximize their surface area and therefore provide higher mRNA binding capacity. In this example, a set of 2 CellBC beads with colours Col1 and Col2 (out of e.g. 12 features) are in a well with 3 ColourBC beads with colour combinations B, BG and GYR (out of e.g. 16). As in Figure 3, the synthetic, (photo)cleavable polyA ending oligos with ColourBCs are released by UV light or chemically and the released oligos will hybridize to the poly-T oligos of both CellBC beads. The bound cellular polyA RNA will also get unique barcodes from these same 2 CellBC beads, and sequence data from these can be merged. In case the chip contains wells with the Col1 CellBC bead with the same 3 ColourBC beads and wells with Col2 CellBC also with the same 3 ColourBC beads, the mapping of the data from these wells spatially is challenging even if the bead specific CellBC sequences are distinct. In such case the transcriptome profile similarities between the cell barcodes can be utilized in spatial prediction. Furthermore, the ColourBC beads can have multiple alternative sequence tags for the same colour or visible feature, to increase the number of distinct combinations in the sequence level, e.g. visually identical blue beads having either B1 (GCACTA), B2 (TCTAGG), B3 (CATTGT) sequences, all independently tagging the blue beads.

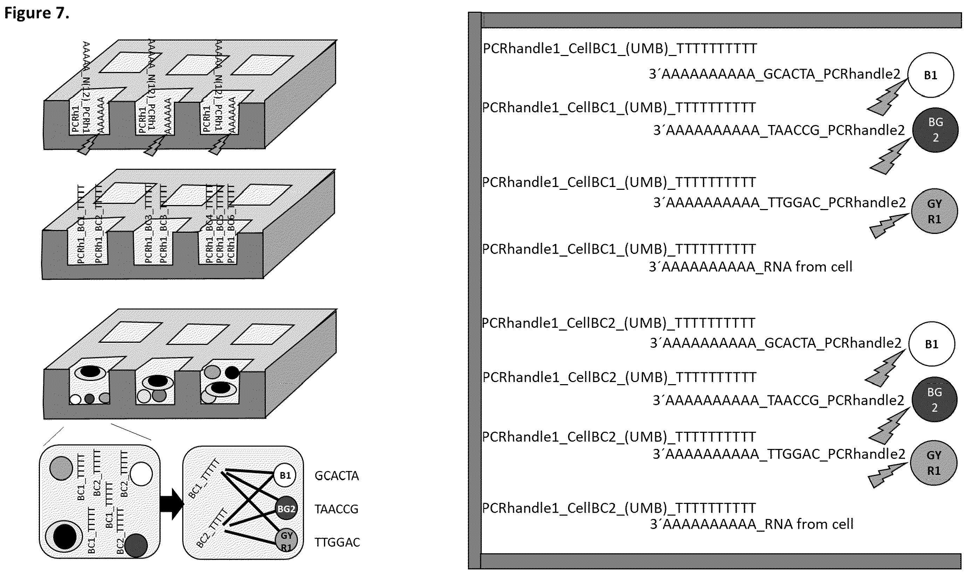

**Figure 7****.** Alternative way to use the ColourBC beads in wells that have the CellBC oligos immobilized on the well bottom and/or wall surface, instead of the bead surface. Random sets of CellBC1-CellBC(n) sequences, unique to each well and at least one per well, can be created in several ways, e.g. by bridge amplification. First a PCRhandle1 oligo (SEQ ID NO: 31) together with a separate cleavable polyA oligo (SEQ ID NO:32), are immobilized densely to all wells and used then as primers in bridge amplification PCR for a template oligo that contains a reverse complement sequence of PCRhandle1, a random CellBC sequence e.g. N(12) or NNNNNNNNNNNN, and polyA sequence (SEQ ID NO:33). Adding the template oligo to the bridge amplification PCR reaction in very diluted concentration allows local clustering of only one or a few different CellBC sequences per well, creating therefore unique set of amplified cell barcodes per well. After the PCR, the polyT oligo is cleaved and removed, leaving only single stranded CellBC containing mRNA capture oligos to the well surface. After this, the well array can be loaded and imaged with the cells and random sets of ColourBC beads with an oligo comprising PCRhandle2, a feature tagging barcode and a polyA. This cleavable oligo from the ColourBC beads will bind to the immobilized CellBC oligos along with the mRNA from the lysed cells, providing spatial information for each cell and well, as in previous Figures.

### DETAILED DESCRIPTION OF EMBODIMENTS

The constructs, compositions and methods described in detail below provides means to generate location data for a single cell of a biological sample, such as a tissue section, among single-cell transcriptome data, on the scale of tens of thousands of cells per experiment. The efficiency of any number of diagnostic techniques and applications for assaying various disease states can be enhanced by use of the compositions described herein. The methods and compositions described herein greatly expand the power of single-cell phenotyping by combining location information and transcripts from the same single cells at an unprecedented scale.

The definitions below are provided for clarity in describing the constructs and compositions herein and are not intended to limit the claimed invention.

As used herein, the term "construct" refers to a chemically synthesized and optionally genetically engineered assembly that comprises a bead attached (preferably covalently) to at least one oligonucleotide sequence by a linker. Each oligonucleotide sequence preferably comprises several functional elements: an amplification handle sequence; a barcode sequence, an optional unique molecular barcode (UMB) sequence that is positioned adjacent to the barcode on its 5' or 3' end, and an anchor sequence for hybridizing to a capture sequence that comprises a sequence complementary to the anchor.

The term "bead" refers herein to a solid support which may encompass any type of solid, porous, or hollow sphere, ball, bearing, cylinder, or other similar configuration composed of plastic, ceramic, metal, or polymeric material (e.g., hydrogel) onto which a nucleic acid can be immobilized (e.g., covalently or non-covalently). The bead may comprise a discrete particle that may be spherical (e.g., microspheres) or have a non-spherical or irregular shape, such as cubic, cuboid, pyramidal, cylindrical, conical, oblong, or disc-shaped, and the like.

The term "amplification handle" generally refers to a functional component of the oligonucleotide sequence in the construct of the present disclosure that provides an annealing site for amplification of the oligonucleotide sequence. In some embodiments, when present in first or additional oligonucleotide sequences, the amplification handle can be the same or different, depending upon the techniques intended to be used for amplification.

The term "barcode" (BC) generally describes a defined oligonucleotide sequence, which when it is a functional element of the construct of the present disclosure, can be used for identifying a particular cell or well in a substrate.

The term "unique molecular barcode" (UMB) generally refers to a random nucleotide, which when it is a functional element of the construct of the present disclosure, is specific for that construct. The UMB permits identification of amplification duplicates of the construct oligonucleotide sequence with which it is associated.

The "linker" refers to any moiety used to attach or associate the bead to the oligonucleotide sequence portion of the constructs. Thus in one embodiment, the linker is a covalent bond. In another embodiment, the linker is a non-covalent bond. The linkers used in the constructs of the compositions and methods are in one embodiment cleavable. The linkers used in the constructs of the compositions and methods are in one embodiment non-cleavable. Without limitation, in one embodiment, the linker is a cleavable linker, e.g., disulfide bond or photocleavable bond. In an example, the parts of the construct are linked to each other via a streptavidin-biotin linkage. In another example, the linker comprises a complex of biotin bound to the construct oligonucleotide sequence by a disulfide bond, with streptavidin fused and coated to the bead. In another embodiment, the biotin is coated to the bead and the streptavidin is fused to the construct oligonucleotide sequence. In another embodiment the bead surface is carboxylated and the oligonucleotide sequence is aminated and linked to the bead with EDC reaction. Photocleavable amino group can be used to enable photocleavage with UV light.

By the term "substrate" is preferably meant a slide, a multi-well plate or a chip. The substrates are conventional and can be glass, polymer or of any conventional materials suitable for the particular assay or diagnostic protocols. The substrates can comprise a matrix of microwells for positioning cells from a biological sample. The substrate can be also a membrane with well structure and with microscopic pores in the well bottoms, to enable easier washing and bead loading.

The terms "visible" and "visually detectable" are used herein to refer to a feature of the present constructs or an entity coupled to said construct that are observable by visual inspection, with or without prior illumination, or chemical or enzymatic activation. In an embodiment of the invention, such visually detectable features may refer to color, size or shape of the construct. Alternatively, such visually detectable features can absorb and emit light in a region of the spectrum ranging from about 400 to about 800 nm. For purposes of the invention, the detection of the present construct by its visible properties means that the construct is preferably observed, with or without illumination or chemical or enzymatic activation, with the aid of an optically based detection device, including, without limitation, absorption spectrophotometers, transmission light microscopes, fluorescence microscopes, digital cameras and scanners.

As used herein, a "biological sample" as used in the methods described herein refers to a naturally-occurring sample or deliberately prepared sample or library containing one or more cells. In one embodiment, a sample contains a population of cells or cell fragments, including without limitation cell membrane components, exosomes, and sub-cellular components. The cells may be a homogenous population of cells, such as isolated cells of a particular type, or a mixture of different cell types, such as from a biological fluid or tissue of a human or mammalian or other species subject. Still other samples for use in the methods and with the compositions include, without limitation, blood samples, including serum, plasma, whole blood, and peripheral blood, saliva, and urine. In one embodiment, the sample is a "tissue section" referring to a piece of tissue that has been obtained from a subject, fixed, sectioned, and mounted on a planar surface, e.g., a microscope slide. Tissue sections can also be classified as "planar cellular samples" referring to a substantially planar, i.e., two dimensional, material that contains cells. A planar cellular sample can be made by, e.g., cutting a three dimensional tissue biopsy that contains cells into sections and mounting the sections onto a planar surface. The cells may be fixed using any number of reagents including formalin, ethanol, methanol, DSP, paraformaldehyde, methanol:acetic acid and other similar fixing or tissue/RNA stabilizing reagents.

The constructs and methods described herein can be used to analyze cells from a subject to determine, for example, what kind of cell types exists in the tissue, in which locations and proportions they exist, whether the cell is normal or not, or to determine whether the cells are responding to a treatment. In one embodiment, the method may be employed to determine the degree of dysplasia in cancer cells. In these embodiments, the cells may be a sample from a multicellular organism. A biological sample may be isolated from an individual, e.g., from a soft tissue. In particular cases, the method may be used to distinguish different types of cancer cells in fresh frozen, cryopreserved and methanol fixed, or formalin fixed paraffin embedded (FFPE) samples. In alternative embodiments, the method described above can be practiced on planar cellular samples that have been fixed in other ways.

In an embodiment, the present invention is directed to a construct for detecting nucleic acid targets, said construct comprising a bead, preferably a magnetic bead, wherein said bead comprises one or several visually detectable features and/or said bead is coupled to one or several visually detectable entities, and wherein said bead comprises an oligonucleotide, and said oligonucleotide comprises a) an amplification handle sequence, b) a barcode sequence specific to one or several of said visually detectable features and/or entities and c) a 3' terminal capture or anchor sequence, and wherein said oligonucleotide is arranged to be detachable from said bead.

In a preferred embodiment, said visually detectable feature is the size of the bead, color of the bead, shape of the bead or a combination thereof.

In another preferred embodiment, said oligonucleotide is linked to said bead via a cleavable linkage.

In another preferred embodiment, said cleavable linkage is a photocleavable, streptavidin-biotin, or carboxylate-amine linkage.

In another preferred embodiment, said bead is coupled to said one or several visually detectable entities via a streptavidin-biotin or carboxylate-amine linkage or via any other linkage.

In another preferred embodiment, said one or several visually detectable entities comprise a fluorescent label, preferably, without limitation, selected from the group consisting of: cyanine dyes, Texas Red dyes and fluorescein amidite dyes.

In another preferred embodiment, said bead is further coupled to an antigen or an antibody.

In another preferred embodiment, said amplification handle sequence is complementary to a PCR primer comprising a next generation sequencing compatible adapter sequence.

In another preferred embodiment, said construct comprises a second oligonucleotide coupled to said bead, said second oligonucleotide comprising a) a second amplification handle sequence preferably complementary to a PCR primer comprising a next generation sequencing compatible adapter sequence, b) a barcode sequence specific to one or several of visually detectable features and/or entities of the bead, c) a barcode sequence specific to the bead, d) an optional unique molecular barcode sequence, and e) a terminal poly-T sequence.

In another embodiment, the present invention is directed to a composition comprising the construct as defined above.

In a preferred embodiment, said composition comprises a mixture of several constructs providing a mixture of distinct visually detectable features or entities.

In a preferred embodiment, said mixture of several constructs comprises at least 2, 3, 4, 5 or 6, preferably at least 16, separate visually detectable features and/or entities.

In a preferred embodiment, said composition comprises multiplicity of said constructs having at least 15 variable sets of visually detectable features such as fluorescent labels coupled to said constructs, wherein
in the first construct said fluorescent label consists of a first fluorescent dye;
in the second construct said fluorescent label consists of a second fluorescent dye;
in the third construct said fluorescent label consists of a third fluorescent dye;
in the fourth construct said fluorescent label consists of a fourth fluorescent dye;
in the fifth construct said fluorescent label consists of a mix of the first and second fluorescent dyes;
in the sixth construct said fluorescent label consists of a mix of the first and third fluorescent dyes;
in the seventh construct said fluorescent label consists of a mix of the first and fourth fluorescent dyes;
in the eighth construct said fluorescent label consists of a mix of the second and third fluorescent dyes;
in the ninth construct said fluorescent label consists of a mix of the second and fourth fluorescent dyes;
in the tenth construct said fluorescent label consists of a mix of the third and fourth fluorescent dyes;
in the eleventh construct said fluorescent label consists of a mix of the first, second and third fluorescent dyes;
in the twelfth construct said fluorescent label consists of a mix of the first, third and fourth fluorescent dyes;
in the thirteenth construct said fluorescent label consists of a mix of the second, third and fourth fluorescent dyes;
in the fourteenth construct said fluorescent label consists of a mix of the first, second and fourth fluorescent dyes; and
in the fifteenth construct said fluorescent label consists of a mix of the first, second, third and fourth fluorescent dyes.

In another preferred embodiment, said first fluorescent dye is Cy5, the second fluorescent dye is Cy3, the third fluorescent dye is Atto425 and the fourth fluorescent dye is FAM.

In another preferred embodiment, different sizes, shapes or visible (non-fluorescent) colours are used in beads, with or without fluorescent dyes, to create multiple distinct bead constructs.

In another preferred embodiment, said composition further comprises a second construct (i.e. a CellBC bead) comprising a bead coupled to a second oligonucleotide, said second oligonucleotide comprising a) a second amplification handle sequence, b) a barcode sequence specific to the bead, c) an optional unique molecular barcode sequence, and d) a second anchor sequence, preferably a poly-T sequence, for hybridizing to a complementary sequence.

In another preferred embodiment, said second oligonucleotide comprising a) a second amplification handle sequence, b) a barcode sequence specific to the bead, c) an optional unique molecular barcode sequence, and d) a second anchor sequence, preferably a poly-T sequence, can be attached or immobilized directly to a bottom and/or wall of a well in a substrate (see, e.g., Figure 7).

In another embodiment, the present invention is directed to a use of the construct or the composition as defined above for identifying mRNA sequencing products originating from a single cell.

In another embodiment, the present invention is directed to a method for profiling biological samples on a single cell level, the method comprising the step of:
- loading the construct as defined in the present disclosure or the composition as defined in the present disclosure with a biological sample together or sequentially in any order to a substrate comprising wells,
- photographing or scanning the wells in said substrate with a microscope, preferably a light microscope or a fluorescence microscope, before and/or after loading the biological sample to said substrate; and
- optionally determining the location or coordinate of a specific well having a distinct visually detectable feature and/or entity or combination thereof in the loaded substrate.

The determination of the location or coordinate of the wells/samples is achieved by examining the photograph or the scanned picture obtained in the method as the visual properties of the constructs of the present invention are visible in the photograph or the scanned picture and thus the location of each type of constructs or mixtures thereof can be determined. In a preferred embodiment, the wells/samples in a substrate are photographed or scanned only once in the present method, i.e the present method does not require taking consecutive photos or scannings of the substrate with changing sets of labels, such as labelled oligonucleotide probes or labelled beads.

In a preferred embodiment, the method comprises the steps of:
- loading the construct or the composition as defined in the present invention with a biological sample together or sequentially in any order to a substrate comprising microwells,
- photographing or scanning the loaded microwells in said substrate with a microscope, preferably a light microscope or a fluorescence microscope,
- subjecting the samples in the microwells to conditions supporting cell lysis, nucleic acid release and then optionally to reverse transcriptase reaction,
- amplifying the released nucleic acids or the reverse transcriptase reaction products in order to facilitate the preparation of a sequencing library of the amplified products, wherein the amplification products contain barcode sequences specific to one or several of visually detectable features and/or entities of the constructs,
- preparing a sequencing library and sequencing the library; and
- optionally identifying the specific microwell location from which each of sequenced products originated by analyzing the sequencing data with barcode information specific to visually detectable features and/or entities of the constructs in order to link a certain visually detectable feature and/or entity or combination thereof to a sequenced product, and determining the location of a specific microwell having the corresponding visually detectable feature and/or entity or combination thereof in the loaded substrate as photographed or scanned in the method.

The invention thus can be used for creating a single-cell sequencing library by merging uniquely barcoded mRNA capturing microbeads with a single cell in a micro-well; lysing the cell thereby capturing the mRNA on the mRNA capturing oligonucleotides on the microbead; performing a reverse transcription reaction in said micro-well to convert the cells' mRNA to first strand cDNA containing said unique barcodes that record the location of each mRNA at the micro-well plate and subsequently sequencing the cDNAs produced.

In a preferred embodiment, the method comprises the steps of:
i) providing multiple constructs according to the present invention, wherein said constructs comprise a plurality of first beads, wherein said first bead has a specific combination of visually detectable features and/or entities such as colour, labels, a specific bead size or shape, and wherein said first bead is coupled to a first oligonucleotide by a photocleavable linker, said first oligonucleotide comprising a) an amplification handle sequence, b) a barcode sequence specific to one or several of said visually detectable features and/or entities and c) a terminal sequence comprising a poly-A sequence;
ii) loading randomly multiple constructs obtained in step i) to microwells, along with capturing constructs comprising a magnetic bead comprising a second oligonucleotide, said second oligonucleotide comprising a) a second amplification handle sequence, b) a barcode sequence specific to the bead, c) an optional unique molecular barcode sequence, and d) a second terminal sequence comprising a poly-T sequence, and a biological sample comprising single cells or alternatively placing cells or a tissue section on a glass slide on to a substrate comprising microwells; and
iii) photographing or scanning the loaded substrate with fluorescence or light microscope.

In a more preferred embodiment, the method further comprises the steps of:
iv) sealing or compartmentalizing the microwells of the loaded substrate with, e.g., a semipermeable membrane or another porous material, oil or gel layer, or the glass slide carrying the cells or tissues on the surface;
v) lysing the cells to release mRNA within microwells with freezing or heating steps, or with a lysis buffer added into the microwells through the membrane or porous sealing on the top or bottom of the microwells, or lysing agent carried by the gel sealing;
vi) detaching the first oligonucleotide from the first bead by subjecting the substrate to UV light, and subjecting the samples in microwells to conditions supporting annealing of poly-A and poly-T sequences of the released mRNA and the second oligonucleotide as well as the first and second oligonucleotides with each other in order to bind said mRNA and first oligonucleotide to the magnetic bead of the capturing construct;
vii) collecting and pooling the samples from the microwells and separating the magnetic bead with attached mRNAs and the bound first and second oligonucleotides from the rest of the sample contents with a magnet and centrifugation facilitated washing steps;
viii) subjecting the separated magnetic beads with attached mRNAs, and the first and second oligonucleotides to a reverse transcriptase reaction with a template switching oligonucleotide comprising a third amplification handle sequence; alternatively the reverse transcription can be performed directly on the microwells before collecting and pooling in step vii);
ix) subjecting the products of the reverse transcriptase reaction to a PCR reaction with primers binding to the first, second and third amplification handle sequence or a complement thereof to produce PCR reaction products which are not attached to the magnetic beads;
x) separating the PCR products from the magnetic beads;
xi) optionally size-selecting the PCR products into a first group of sequences having the length of under about 200 bp and a second group having the length of above about 200 bp;
xii) introducing sequencing index and adapter sequences (preferably with appropriate tagmenting, fragmenting, end-repair, A-tailing, ligation, or PCR based methods when necessary) and sequencing the PCR products, optionally in the groups obtained in step xi);
xiii) identifying the mRNA products of a single cell by analyzing the sequencing data, preferably by comparing the combinations of the barcode sequences specific to a visually detectable feature and/or entity or combination thereof derived from the first oligonucleotide and the barcode sequence specific to the magnetic bead derived from the second oligonucleotide and/or optionally the unique molecular barcode sequence derived from the second oligonucleotide, present in the same sequenced PCR product, to a sequenced PCR product comprising a combination of a mRNA sequence and the barcode sequence specific to the bead derived from the second oligonucleotide and/or optionally the unique molecular barcode sequence derived from the second oligonucleotide;
xiv) optionally locating the single cell or tissue location by comparing the combinations of the barcode sequences specific to a visually detectable feature and/or entity or a combination thereof derived from the first oligonucleotide and the photograph or scan of the loaded substrate taken with fluorescence or light microscope in step iii).

In another preferred embodiment, the method comprises the steps of:
i) providing multiple constructs comprising first and second oligonucleotides as defined above, wherein said constructs comprise a plurality of first beads, wherein said first bead has a specific combination of visually detectable features and/or entities such as colour, labels, a specific bead size or shape, and wherein said first bead is coupled to a first oligonucleotide by a photocleavable linker, said first oligonucleotide comprising a) an amplification handle sequence, b) a barcode sequence specific to one or several of said visually detectable features and/or entities and c) a terminal sequence comprising a poly-A sequence, and wherein said first bead further comprises a plurality of second oligonucleotides coupled to said bead, said second oligonucleotide comprising a) a second amplification handle sequence complementary to a PCR primer comprising a next generation sequencing compatible adapter sequence, b) a barcode sequence specific to one or several of visually detectable features and/or entities of the bead, c) a barcode sequence specific to the bead, d) an optional unique molecular barcode sequence, and e) a terminal poly-T sequence;
ii) loading randomly multiple constructs obtained in step i) to microwells and a biological sample comprising single cells or alternatively placing cells or a tissue section on a glass slide on to a substrate comprising microwells; and
iii) photographing or scanning the loaded substrate with fluorescence or light microscope.

In a preferred embodiment of the invention, the detection of the construct of the present invention is not dependent on addition of oligonucleotide probe(s) to said wells/samples, said probe(s) comprising an optically readable label and having complementary sequence(s) to said first and/or second oligonucleotide.

While the following examples are illustrative of the principles of the present invention in one or more particular applications, it will be apparent to those of ordinary skill in the art that numerous modifications in form, usage and details of implementation can be made without the exercise of inventive faculty, and without departing from the principles and concepts of the invention. Accordingly, it is not intended that the invention be limited, except as by the claims set forth below.

The verbs "to comprise" and "to include" are used in this document as open limitations that neither exclude nor require the existence of also un-recited features. The features recited in depending claims are mutually freely combinable unless otherwise explicitly stated. Furthermore, it is to be understood that the use of "a" or "an", that is, a singular form, throughout this document does not exclude a plurality.

Reference throughout this specification to one embodiment or an embodiment means that a particular feature, structure, or characteristic described in connection with the embodiment is included in at least one embodiment of the present invention. Thus, appearances of the phrases "in a preferred embodiment" or "in an embodiment" in various places throughout this specification are not necessarily all referring to the same embodiment. Where reference is made to a numerical value using a term such as, for example, about or substantially, the exact numerical value is also disclosed.

### EXPERIMENTAL SECTION

The following examples disclose four versions of the present method to create visual bead barcoded coordinates for the spatial RNAseq analysis of single cells or tissues. These examples should not be construed to encompass any and all variations that become evident as a result of the teaching provided herein.

### EXAMPLE 1

### Design and Validation of Colour Barcode Microbeads

16 different Colour Barcode Microbead constructs were designed, where 2.8µm diameter sized magnetic and streptavidin coated Dynabeads (M-280, ThermoFisher Scientific, catalog number 11205D), were used as bead backbones. For single or multicolour staining of the beads, four short 5' biotinylated oligos with stretch of 10 C's as a spacer and each with different fluorescent blue (Atto425), green (FAM), yellow (Cy3) and red (Cy5) labels at 3' end were ordered from Integrated DNA Technologies (SEQ ID NO: 22-25). Sequence of the staining oligos is irrelevant and can be replaced by other sequences or spacer molecules. Another set of 16 different Colour barcode tagging oligos with photocleavable biotin (PCbio) (released by UV exposure at 300-350nm wavelengths) in the 5' end were designed (SEQ ID NO: 1-16) with the following characteristics: a 5' end generic amplification handle (PCR handle2) for next-generation sequencing library preparation, a unique barcode sequence specific for each bead colour construct, and a polyA stretch at the 3' end (Fig 4 and table of Fig 5). 16 batches of beads were prepared, each with one of the 16 Colour barcode oligos. 15 of the batches were stained also with a different combination of 1, 2, 3 or 4 of the fluorescently labelled biotinylated oligos. The 16th batch of beads was used as non-fluorescent, labeled only with the Colour barcode oligo.

Streptavidin Dynabeads have a capacity of binding ~200pmols of biotinylated DNA oligos per mg of beads and the labelled and unlabelled oligos were added on beads in 1:1 to 1:10 ratios depending on each fluorochrome's signal intensities. After incubation of 15 min at RT in rolling tubes the beads were washed thoroughly 5 times with PBS and pelleted by magnet in each washing round. 1X Dynabead binding and washing (B&W) Buffer was used in these steps (5 mM Tris-HCl (pH 7.5), 0.5 mM EDTA, 1 M NaCl). Fluorescence intensities and bead integrities were checked with Zeiss AxioImager microscope. The selection of labels, staining intensities and exposure times need to be adjusted at each user lab to be compatible with their fluorescent microscope specifications. Proper binding and release of the Colour barcode oligo was tested from each 16 bead batches by incubating them with commercial Cell barcoding beads (MACOSKO 2011-10 B, ChemGenes, USA, hereafter called CellBC or Cell barcoding beads) with 10 min UV exposure (Bio-Rad ChemiDoc Imaging system) to release the oligo, followed by 20min hybridization of polyA to polyT, subsequent separation of non-magnetic CellBC beads from the magnetic Colour barcode beads with magnet, 3 rounds of thorough SSC buffer (Sigma Aldrich, catalog number S6639) washing and centrifugation steps of CellBC beads, PCR amplification at bulk reaction (SEQ ID NO: 19 and 26 ) and gel electrophoresis detection of the correct sized (~180bp) product. After the quality check, the prepared 16 bead batches were mixed in equal proportions and this stock bead mix was stored at +4 in Tris-PBS buffer and protected from light.

### Microwell array loading

Aliquot of ColourBC bead stock mix containing ~100 000 beads was pelleted with magnet and washed once, and suspended to 200µl PBS with thorough but gentle pipet mixing at least 20 times, and then loaded randomly to microwell array area 1cm x 1cm consisting of 20 000 microwells of the size of 20µm diameter and 20µm depth (customized geometry designed and fabricated of polydimethyl sulfoxide (PDMS) but otherwise functionalized according to the Seq-Well protocol by Gierahn et al 2017). Removable silicone frames around the array area and a glass cover slip slide on top of the buffer was used to help in equal loading efficiency and prevention of buffer evaporation. Flat magnet was placed under the array slide to fasten the bead loading. During the 10 min loading the magnet was carefully removed twice and the chip shaked gently horizontally to enhance the remaining beads between the microwells to settle down to the wells. Loading quality was also checked under light microscope. Extra buffer was removed, keeping the slide array still on magnet, and slides were let to dry for at least 30min protected from light. Loaded and dried arrays were stored in covered petri dishes at +4°C protected from light, until the next step.

### Image analysis for coordinates

Fluorescent microscope scanner Zeiss AxioImager was used to scan the whole array area for the 4 fluorescent colours and their combinations used on beads, and the associated Zen 2.3 Lite program to form a single stitched large image of the area. Image was saved as 4 fluorescent and 1 light microscope channels in a stacked multilayer image format. Image analysis and machine learning algorithm was created, trained and validated with a specific machine learning software designed for microscope image analysis. Algorithm was trained to recognize the 16 different classes of beads (constructs) based on their colour combinations, to recognize the 20µm diameter sized microwells, to count the number of each type of these bead classes within each microwell, and to give a X-Y positioned coordinate on array for each such feature composition. Nearly all wells loaded with this loading protocol contained from 1 to over 10 beads per well, with most microwells having 3-8 beads as predicted by Poisson distribution. Out of the 16 possible features, total of theoretical 65535 combinations are possible (Fig 2), and the AI algorithm mapped and recorded the wells with unique composition of bead features. List of non-unique combinations were also marked to the map.

Unless proceeding immediately to the next step, the pre-loaded and image coordinated slides can be prepared in stock beforehand and stored at +4°C dry with magnet underneath if handled, and protected from light and dust.

### Single cell RNAseq experiment with Seq-Well protocol combined to the present method

Pre-loaded and imaged chips were wetted for 2 hours with PBS, with magnet under the chip and light protected. 20 000 commercially available Cell barcoding (CellBC) beads, custom synthetized on 10µm diameter polystyrene beads (ChemGenes, MACOSKO-2011-10 B with SEQ ID NO: 17, synthesis described in Macosko et al 2015) were prewashed and loaded in 200µl volume of bead loading buffer (recipe on Gierahn et al 2017) on the desired microwell area with gentle horizontal shaking for 30 min, and using silicon frames and light protection. After this 5000 peripheral blood mononuclear cells in 200µl RPMI medium were added and cells let to settle in similar manner for 30 min. The wells were sealed with semipermeable membrane and cells lysed with lysis buffer, adapted from Seq-Well protocol (Gierahn et al 2017). 5 min UV light exposure was used to release the photocleavable oligos from the Colour barcode beads. After the 20min incubation the membrane was removed, CellBC beads were collected and washed, 1st strand cDNA synthesis was performed with reverse transcription with template switching oligo (SEQ ID NO: 18) followed by ExoI treatment, PCR amplification of full length cDNA (SEQ ID NO: 19) and tagmentation based Illumina library preparation (SEQ ID NO: 20 and standard Illumina Nextera i7 indexing primer) with appropriate washing, purification and quantitation steps, following the DropSeq (Macosko et al 2015) and Seq-Well (Gierahn et al 2017) protocols. Size selection of <200bp fragments and separate library preparation (with SEQ ID NO:26 and SEQ ID NO:27) of the Colour barcodes linked to Cell barcodes, followed the CITE-seq protocol for ADT (antibody determining tag) libraries (Stoeckius et al 2017).

### Sequencing and data analysis

The libraries were sequenced with Illumina NextSeq500 platform and High Output 75 cycle sequencing kit. Custom read 1 sequencing primer (SEQ ID NO: 21) was used to sequence 20bp for read 1 covering the 12bp cell barcode and 8bp molecular barcode (UMB) derived from the CellBC beads. Standard Illumina read 2 sequencing primers included in the sequencing cartridge produced 55bp reads of the gene sequences derived from captured mRNAs by the CellBC beads, as well as the colour bead barcodes derived from the UV-detached feature oligos that were also captured by the CellBC beads. Processing of raw fastq files followed the publicly available Drop-Seq data analysis pipeline (Macosko et al 2015) and, for the colour barcode, the CITE-seq pipeline (Stoeckius et al 2017). A combined gene expression matrix was created from the counts of transcripts aligned to the reference genome and the colour barcode counts linking to the same cell barcode. This matrix was further matched with the visual map of the spatial X-Y coordinates of the cells and beads in the wells, created earlier with the machine learning algorithm from the microscope scanner image.

### EXAMPLE 2

Another variation of the protocol described in Example 1 was tested with different sizes (3, 6, 8 or 10um) and visual colours of the beads, combined to just a single fluorescence label FAM. Such set of beads is easier to use with large variety of end-user microscopes as they need less optimization compared to multi-fluorescence excitation, emission and filter sets, and possible autofluorescence limitations of some beads. Beads in various sizes, colours and surface coatings or functionalizations are available from many vendors (e.g. Spherotech, Bangs Laboratories, PolySciences, Abvigen). When carboxylated beads were used instead of streptavidin coating, the FAM labelled staining oligo was ordered with 5' amine group instead of biotin, and respectively the Colour barcode oligos were ordered with photocleavable 5' end amine group (GeneLink). EDC activation was used to form amide bond between the carboxyl groups on beads and the primary amine of the oligo, following the instructions of the reagent provider (ThermoFisher Scientific, catalog number 22980). In case of non-magnetic beads, they were magnetized with aminated magnetic nanoparticles (Merck Life Science, catalog number 747327) in the same EDC reaction. The linked beads were washed thoroughly at least 3 times to remove the unbound oligos and labels, before mixing the bead batches. Otherwise the protocol was similar to Example 1, the loaded beads were scanned with AxioImager fluorescence microscope equipped with a colour camera and the stitched colour image stacked with the green fluorescence image (for FAM label) were analyzed with a machine learning algorithm that was taught to recognize the following 20 classes of beads and recognize and count them from each microwell. Random sets of beads from the pool of 20 bead types produces over a million unique combinations of bead types per well (https://stattrek.com/online-calculator/combinations-permutations.aspx) as the sum of all possible 1, 2, 3, 4, 5, 6, 7, 8, 9, 10, 11, 12, 13, 14, 15, 16, 17, 18, 19 and 20 bead combinations out of 20 bead types totals to 20+190+1140+4845+15504+38760+77520+125970+167960+184756+167960+125970+ 77520+38760+15504+4845+1140+190+20+1=1048575 possible combinations per well. For example, in an array with 10000 wells and loading with 80000 beads randomly in Poisson distribution, the proportion of non-informative/non-unique wells with 0, 1 or 2 beads only can be kept in less than 2%.

20 bead types created in example 2:
Blue-3um-non-fluorescent
Blue-6um-non-fluorescent
Yellow-3um-non-fluorescent
Yellow-6um-non-fluorescent
Red-3um-non-fluorescent
Red-6um-non-fluorescent
Non-coloured-3um-non-fluorescent
Non-coloured -6um-non-fluorescent
Non-coloured -8um-non-fluorescent
Non-coloured -10um-non-fluorescent
Blue-3um-FAM
Blue-6um-FAM
Yellow-3um-FAM
Yellow-6um-FAM
Red-3um-FAM
Red-6um-FAM
Non-coloured -3um-FAM
Non-coloured -6um-FAM
Non-coloured -8um-FAM
Non-coloured -10um-FAM

### EXAMPLE 3

Another variation of the protocol is the method without the commercial Cell barcode beads, but instead the Cell barcode oligo is carried by the coloured or other visibly featured beads, along with the ColourBC oligos (see Figure 4). Optionally, the new CellBC oligo carried on the featured beads carries also an additional ColourBC sequence matching with the colour or feature combination of the beads (SEQ ID NO: 28). It is essential, though, that the featured beads also carry a separate cleavable ColourBC oligo that is released and captured randomly by all beads in a given well. The unique CellBC oligo sequence was created to each bead batch with biotinylated backbone oligo consisting PCRhandle1 and optional ColourBC. To create them, the split-and-pool synthesis of 12bp bead specific CellBC, 8bp totally random molecular barcode and 30bp poly-T sequence was adapted from the protocol described in Macosko et al 2015. After this, the ColourBC oligos with poly-A end, and photocleavable biotin on 5' and identical to example 1, were attached to the bead batches, washed 5 times, and then all bead featured batches were mixed and used in the experiments on microwells. Beads were loaded and imaged as in Example 1, but no commercial CellBC beads were added in following steps. Cell or tissue loading and further analysis followed otherwise the examples 1 and 4. Multiple beads per well created multiple cell barcodes per well, but as the colour or feature combination was unique, it allowed matching of multiple Cell barcodes to a single unique ColourBC combination in each well, and this was allowed in the bioinformatics analysis pipeline with this method version.

### EXAMPLE 4

Another variation of the method is to use the bead barcoded microwells for direct spatial capture of the RNA from cryopreserved or formalin fixed paraffin embedded (FFPE) tissue sections, instead of dissociated cells in suspension. For this, snap frozen and TissueTek OCT Compound (Sakura) embedded mouse mammary tumor tissues were sliced with cryomicrotome to 10µm thick sections on standard microscope glass slide, and kept in -80°C until used. Sections were fixed with methanol and stained with standard hematoxylin-eosin (HE) staining protocol, followed by light microscope image scan to visualize the tissue structure. A previously prepared microwell slide with ColourBC beads pre-loaded and fluorescently imaged (as described in examples 1 and 3) was kept on magnet and wetted with cell permeabilization buffer (0.1% pepsin in 0.1 N HCl). The wetted array was placed on a humidified table-top chamber to prevent evaporation of the small liquid volumes from the microwells in the following steps. Keeping the slides within the chamber, the excess lysis buffer was removed from the top of the slide followed by immediate placing of the tissue section slide on top of it so that the lysis buffer filled wells and the tissue got in contact. Thin piece of metal taped on the backside of the tissue slide helped to hold it tightly and still in place with the power of the magnet under the microwell slide. Alternatively, a mechanical clip could be used. Cells were let to lyse and mRNA to hybridize on the beads for 15 min at room temperature. 5 min UV exposure was introduced in the beginning as in the Example 1 to release the Colour barcode oligos from the beads and hybridize them with the Cell barcoding oligos along with the mRNA from the lysing cells. After this, the tissue slide was quickly moved and the microwell array, while still on magnet, was immediately washed 4 times with SSC buffer to remove the excess of non-hybridized RNA and the UV-released oligos. Beads were collected from the microwell slide by placing the slide upside down to a slide chamber with SSC buffer and pulling the beads with magnet under the chamber. In case of Example 1 protocol with commercial, non-magnetic CellBC beads a slide centrifugation step was also needed. Beads in buffer were collected to a single Eppendorf tube and pelleted. The following reverse transcription and library preparation steps were done in bulk reaction on the beads, as referred in example 1, following closely the protocols described in Macosko et al 2015, Gierahn et al 2017 and Stoeckius et al 2017. Data analysis was otherwise similar to the one described in example 1, but in addition, the microscope image of the original HE-stained tissue section was overlaid on top of the spatial sequencing information of the gene expression derived from the bead coordinated well positions. Matching the tissue and array images for the overlay was guided by the shape of the sequencing data producing area from the microwell slide and with inbuilt assisting marking spots on both slides recorded with microscope and aligned from the images.

### REFERENCES

Gierahn TM, Wadsworth MH 2nd, Hughes TK, Bryson BD, Butler A, Satija R, Fortune S, Love JC, Shalek AK. Seq-Well: portable, low-cost RNA sequencing of single cells at high throughput. Nat Methods. 2017 Apr;14(4):395-398. doi: 10.1038/nmeth.4179. Epub 2017 Feb 13. PMID: 28192419; PMCID: PMC5376227.

Macosko EZ, Basu A, Satija R, Nemesh J, Shekhar K, Goldman M, Tirosh I, Bialas AR, Kamitaki N, Martersteck EM, Trombetta JJ, Weitz DA, Sanes JR, Shalek AK, Regev A, McCarroll SA. Highly Parallel Genome-wide Expression Profiling of Individual Cells Using Nanoliter Droplets. Cell. 2015 May 21;161(5):1202-1214. doi: 10.1016/j.cell.2015.05.002. PMID: 26000488; PMCID: PMC4481139.

Stoeckius M, Hafemeister C, Stephenson W, Houck-Loomis B, Chattopadhyay PK, Swerdlow H, Satija R, Smibert P. Simultaneous epitope and transcriptome measurement in single cells. Nat Methods. 2017 Sep;14(9):865-868. doi: 10.1038/nmeth.4380. Epub 2017 Jul 31. PMID: 28759029; PMCID: PMC5669064.

List of oligonucleotide sequences and their modifications: 
SEQ ID NO: 1: /5PCbio/CCTTGGCACCCGAGAATTCCA ATCACG
BAAAAAAAAAAAAAAAAAAAAAAAAAAAAAA
 
SEQ ID NO: 2: /5PCbio/CCTTGGCACCCGAGAATTCCA TTAGGC
BAAAAAAAAAAAAAAAAAAAAAAAAAAAAAA
 
SEQ ID NO: 3: /5PCbio/CCTTGGCACCCGAGAATTCCA TGACCA
BAAAAAAAAAAAAAAAAAAAAAAAAAAAAAA
 
SEQ ID NO: 4: /5PCbio/CCTTGGCACCCGAGAATTCCA ACAGTG
BAAAAAAAAAAAAAAAAAAAAAAAAAAAAAA
 
SEQ ID NO: 5: /5PCbio/CCTTGGCACCCGAGAATTCCA GCCAAT
BAAAAAAAAAAAAAAAAAAAAAAAAAAAAAAAAAAAAAA
 
SEQ ID NO: 6: /5PCbio/CCTTGGCACCCGAGAATTCCA CAGATC
BAAAAAAAAAAAAAAAAAAAAAAAAAAAAAAAAAAAA
 
SEQ ID NO: 7: /5PCbio/CCTTGGCACCCGAGAATTCCA ACTTGA
BAAAAAAAAAAAAAAAAAAAAAAAAAAAAAAAAA
 
SEQ ID NO: 8: /5PCbio/CCTTGGCACCCGAGAATTCCA TGAACG
BAAAAAAAAAAAAAAAAAAAAAAAAAAAAAA
 
SEQ ID NO: 9: /5PCbio/CCTTGGCACCCGAGAATTCCA CTGAGA
BAAAAAAAAAAAAAAAAAAAAAAAAAAAAAA
 
SEQ ID NO: 10: /5PCbio/CCTTGGCACCCGAGAATTCCA TAGCTG
BAAAAAAAAAAAAAAAAAAAAAAAAAAAAAAAAAAAAA
 
SEQ ID NO: 11: /5PCbio/CCTTGGCACCCGAGAATTCCA AATACG
BAAAAAAAAAAAAAAAAAAAAAAAAAAAAAA
 
SEQ ID NO: 12: /5PCbio/CCTTGGCACCCGAGAATTCCA TTCCAG
BAAAAAAAAAAAAAAAAAAAAAAAAAAAAAA
 
SEQ ID NO: 13: /5PCbio/CCTTGGCACCCGAGAATTCCA ATCTGT
BAAAAAAAAAAAAAAAAAAAAAAAAAAAAAAAA
 
SEQ ID NO: 14: /5PCbio/CCTTGGCACCCGAGAATTCCA ACTTGA
BAAAAAAAAAAAAAAAAAAAAAAAAAAAAAA
 
SEQ ID NO: 15: /5PCbio/CCTTGGCACCCGAGAATTCCA TAACAG
BAAAAAAAAAAAAAAAAAAAAAAAAAAAAAAAAAAAA
 
SEQ ID NO: 16: /5PCbio/CCTTGGCACCCGAGAATTCCA GATGTG
BAAAAAAAAAAAAAAAAAAAAAAAAAAAAAA
 
SEQ ID NO: 17:
TTTTTTTAAGCAGTGGTATCAACGCAGAGTACBCBCBCBCBCNNNNNNNNTTT
TTTTTTTTTTTTTTTTTTTTTTTTTTTTTT
 (DropSeq Cell barcoding oligo, commercial, where stretches of BC states for cell barcode and N for unique molecular barcode UMB)
SEQ ID NO: 18: AAGCAGTGGTATCAACGCAGAGTGAATrGrGrG (DropSeq-TSO)
SEQ ID NO: 19: AAGCAGTGGTATCAACGCAGAGT (DropSeq-SMART-PCRprimer)

SEQ ID NO: 20:
AATGATACGGCGACCACCGAGATCTACACGCCTGTCCGCGGAAGCAGTGGTATC
AACGCAGAGT*A*C (DropSeq-New-P5-SMART-PCR-hybrid-oligo)

SEQ ID NO: 21: GCCTGTCCGCGGAAGCAGTGGTATCAACGCAGAGTAC (DropSeq-CustomRead1 Sequencing primer)
SEQ ID NO: 22: /Atto425/CCCCCCCCCC/3bio/ (Colour bead fluorescent labelling oligo)
SEQ ID NO: 23: /56-FAM/CCCCCCCCCC/3bio/ (Colour bead fluorescent labelling oligo)
SEQ ID NO: 24: /5Cy3/CCCCCCCCCC/3bio/ (Colour bead fluorescent labelling oligo)
SEQ ID NO: 25: /5Cy5/CCCCCCCCCC/3bio/ (Colour bead fluorescent labelling oligo)
SEQ ID NO: 26: CCTTGGCACCCGAGAATT*C*C (Colour bead PCR-handle 2 amplification primer) 
SEQ ID NO: 27:
CAAGCAGAAGACGGCATACGAGATCGTGATGTGACTGGAGTTCCTTGGCACCCG
AGAATTCCA
(Illumina Small RNA RP11 primer for ColourBC product indexing, here i7 ▪index 1 as example) 
/5bio/TTTTTTTAAGCAGTGGTATCAACGCAGAGTACNNNNNNBCBCBCBCBCBCNN
NNNNNNTTTTTTTTTTTTTTTTTTTTTTTTTTTTTT
(Modified DrpSeq Cell barcoding▪oligo when used in ColourBC beads together with Colour barcoding oligos, including the optional Colour tagging sequence indicated with the first NNNNNN sequence)
SEQ ID NO: 31:
   /5bio/TTTTTTTAAGCAGTGGTATCAACGCAGAGTAC (immobilized PCR-handle oligo for bridge amplification presented in Figure 7)
SEQ ID NO: 32 /5PCbio/AAAAAAAAAAAAAAAAAAAAAAAAAAAAAA (photocleavable immobilized polyA oligo for bridge amplification presented in Figure 7) 
SEQ ID NO: 33

AAAAAAAAAAAAAAAAAAAAAAAAAAAAAANNNNNNNNNNGTACTCTGCGT

TGATACCACTGCTT
(template for bridge amplification in Figure 7, including the N/12 random sequence that creates the well specific CellBC clusters)

## Claims

1. A composition for detecting nucleic acid targets comprising a first construct and a second construct, wherein said first construct comprises a bead, wherein said bead comprises one or several visually detectable features and/or said bead is coupled to one or several visually detectable entities, and wherein said bead comprises an oligonucleotide, and said oligonucleotide comprises a) an amplification handle sequence, b) a barcode sequence specific to one or several of said visually detectable features and/or entities and c) a 3' terminal capture or anchor sequence, and wherein said oligonucleotide is arranged to be detachable from said bead; and
wherein said second construct comprises a bead coupled to a second oligonucleotide, and said second oligonucleotide comprises a) an amplification handle sequence, b) a barcode sequence specific to said bead and c) a second anchor sequence.

2. The composition according to claim 1, wherein said anchor sequence of the second construct is complementary to said 3' terminal capture or anchor sequence of the first construct and is bound to said 3' terminal capture or anchor sequence of the first construct thus forming a combination comprising said first and second constructs.

3. The composition according to claim 1 or 2, wherein said 3' terminal capture or anchor sequence of the first construct is poly-A sequence.

4. The composition according to any one of claims 1-3, wherein said second anchor sequence of the second construct is poly-T sequence.

5. The composition according to any one of claims 1-4, wherein said visually detectable feature is the size of the bead, color, luminescence or fluorescence of the bead, shape of the bead or a combination thereof.

6. The composition according to claim 5, wherein said visually detectable feature is the color of the bead.

7. The composition according to any one of claims 1-6, wherein said bead of the second construct comprises a visually detectable feature such as color and/or shape of the bead.

8. A composition for detecting nucleic acid targets comprising a first construct and a second construct, wherein said first construct comprises a bead, wherein said bead comprises one or several visually detectable features and/or said bead is coupled to one or several visually detectable entities, and wherein said bead comprises an oligonucleotide, and said oligonucleotide comprises a) an amplification handle sequence, b) a barcode sequence specific to one or several of said visually detectable features and/or entities and c) a 3' terminal capture or anchor sequence, and wherein said oligonucleotide is arranged to be detachable from said bead; and
wherein said second construct comprises a second oligonucleotide attached or immobilized directly to a bottom and/or wall of a well in a substrate, and said second oligonucleotide comprises a) an amplification handle sequence, b) a barcode sequence specific to said second construct and c) a second anchor sequence.

9. The composition according to claim 8, wherein said second anchor sequence of the second construct is complementary to said 3' terminal capture or anchor sequence of the first construct and is bound to said 3' terminal capture or anchor sequence of the first construct thus forming a combination comprising said first and second constructs.

10. The composition according to claim 8 or 9, wherein said second anchor sequence of the second construct is poly-T sequence.

11. A well plate or chip comprising the composition according to any one of claims 1-10.

12. A kit comprising the well plate or chip according to claim 11 and a computer readable entity comprising a file of a photograph or scan of said plate or chip with the composition according to any one of claims 1-10.

13. Use of the composition according to any one of claims 1-10 for identifying desired nucleic acid targets or mRNA sequencing products originating from a single cell, position or coordinate.

14. Method for detecting location of biological samples, the method comprising the steps of:
- loading the composition according to any one of claims 1-10 with a biological sample together or sequentially in any order to a substrate comprising wells,
- photographing or scanning the wells in said substrate with a microscope, preferably a light microscope or a fluorescence microscope, before and/or after loading the biological sample to said substrate; and
- optionally determining the location or coordinate of a specific well having a distinct visually detectable feature and/or entity or combination thereof in the loaded substrate.

15. The method according to claim 14 further comprising the steps of:
- subjecting the samples in the wells to conditions supporting cell lysis, nucleic acid release and then to reverse transcriptase reaction,
- amplifying the reverse transcriptase reaction products, wherein the amplification products contain barcode sequences specific to one or several of visually detectable features and/or entities of the constructs,
- preparing a sequencing library from the amplified reverse transcriptase reaction products and sequencing said library; and
- optionally identifying specific well locations from which each of sequenced products originated by analyzing the sequencing data with barcode information specific to visually detectable features and/or entities of the constructs in order to link a certain visually detectable feature and/or entity or combination thereof to a sequenced product, and determining the location of a specific well having the corresponding visually detectable feature and/or entity or combination thereof in the loaded substrate as photographed or scanned in the method.
